# EUROPEAN PATENT SPECIFICATION

(11) **EP 1 067 908 B1**
(45) Date of publication and mention of the grant of the patent: **31.05.2006**
(21) Application number: 99915190.5
(22) Date of filing: 30.03.1999
(51) Int. Cl.: A61K 9/107, A61K 31/335, A61K 47/26, A61K 47/44, A61P 35/00

(54) **TAXANE MICROEMULSIONS**
TAXAN-MIKROEMULSIONEN
MICROEMULSIONS A BASE DE TAXANE

(30) Priority: 01.04.1998 US 80272 P; 01.04.1998 US 80273 P
(43) Date of publication of application: 17.01.2001
(73) Proprietor: JAGOTEC AG, 4132 Muttenz (CH)
(72) Inventor: PARIKH, Indu, Verdun, Quebec H3E 1LII1 (CA); MOUSSA, Iskandar, Beirut (LB); CARRIER, Alain, St-Hubert, Quebec J3Z 1G9 (CA)
(74) Representative: Bassil, Nicholas Charles
(86) International application number: PCT/US1999/007162
(87) International publication number: WO 1999/049848

(56) References cited:
- WO-A-96/02247
- WO-A-98/07434
- US-A- 5 560 931
- LUNDBERG, B.B.: "A Submicron Lipid Emulsion Coated with Amphipathic Polyethylene Glycol for Parenteral Administration of Paclitaxel (Taxol)" J. PHARM. PHARMACOL., vol. 49, 1997, page 16-21 XP002111265
- LIAU-CHU M. ET AL.: "Mechanism of Action of Anaphylactoid Reacitons: Improper Preparation of High-Dose Intravenous Cyclosporine leads to Bolus Infusion of Cremophor EL and Cyclosporine" ANN. PHARMACOTHER., vol. 31, 1997, pages 1287-1291, XP002111266
- MICHAUD L B: "Methods for preventing reactions secondary to Cremophor EL [comment]" ANNALS OF PHARMACOTHERAPY, vol. 31, no. 11, November 1997 (1997-11), pages 1402-1404, XP002099306 ISSN: 1060-0280

## Description

The present invention relates generally to cancer therapeutics. More particularly it is directed to novel pharmaceutical compositions of water insoluble anticancer taxane drugs which include paclitaxel and docetaxel.

### BACKGROUND AND SUMMARY OF THE INVENTION

Paclitaxel is a taxane and a member of the terpenoid family of compounds present in very small quantities in the Taxus brevifolia species such as the pacific Yew tree. These compounds, collectively known as taxoids, taxins or taxanes, have potent anticancer properties in, among others, ovarian cancer, lymphoma, and breast cancer. Because of its poor solubility in water, the current commercial formulation of paclitaxel is prepared by dissolving 6 mg of the drug in one milliliter of a mixture of polyoxyethylated castor oil (Cremophor® (EL) and dehydrated alcohol. The commercially available paclitaxel formulation is for intravenous administration only. There exists no commercial formulation of paclitaxel, which can be administered orally. The commercial injectable formulation is physically unstable especially for treatments requiring long infusion time. The infusate may contain up to 10% each of alcohol and Cremophor® EL. The physical stability of the paclitaxel formulation may be increased by increasing the amounts of Cremophor® EL in the formulation, but may also lead to an increased incidence of adverse reactions. Yet another approach as described in U.S. patent 5,681,846 is to decrease the drug and Cremophor® concentration and increase the alcohol content in the formulation.

An undesirable effect of Cremophor® EL in paclitaxel and other drug formulations is the production of possible anaphylactoid reaction with associated dyspnea, hypotension, angioedema and uticaria. Cremophor® EL is also known to extract plasticizers such as diethylhexyl-phthalate from the polymers commonly used intravenous infusion tubings and infusion bags. These plasticizers are known to promote toxic reactions, such as Adult Respiratory Distress Syndrome (ARDS), in patients which have been exposed to high levels.

Various other methods have been used to increase the water solubility of paclitaxel and other anticancer drugs, for example, by conjugation of the water insoluble drug moiety with water soluble polymers as taught by U.S. patent 5,437,055, WO 97/10849, and WO 97/33552. While WO 94/12031 teaches that a composition of paclitaxel with Cremophor®EL, absolute alcohol and citric acid increases the stability however, no mention is made if the proposed composition increases the solubility of paclitaxel. Others have used liposome preparations as a means of eliminating Cremophor®EL and reducing vehicle toxicity as described by Sharma et al (Pharm. Res. 11:889-896, 1994). An oil-in-water emulsion (U.S. patent 5,616,330) is another approach to preparing Cremophor® free paclitaxel formulation. The latter two formulation approaches have limitations in terms of low degree of drug loading. Yet another approach uses cyclodextrins to make a water-soluble formulation ofpaclitaxel as described in WO 94/26728.

The present invention is based on a strong need for a safer and stable injectable and oral formulation of taxanes such as paclitaxel and docetaxel.

U.S. patent 5,407,683 discloses a composition containing paclitaxel in squalene as solution in absence of a surfactant and then forming a self-emulsifying glass by addition of an aqueous sucrose solution followed by evaporation of water. The resulting glass upon mixing with water forms an emulsion with a particle size in a range of 2 to 10 µm. The preparation of such glass requires the use of undesirable organic solvents, which must be completely removed before medical use.

Quay et al describe a conventional oil-in-water emulsion system (WO 98/30205) consisting of vitamin E as a carrier oil in which a drug may be dissolved, together with polyethyleneglycol and related surfactants. Conventional emulsions have limited shelf life and are often difficult to terminally heat sterilize or even filter sterilize. The particle size of conventional emulsions is usually far greater than microemulsions.

Microemulsions are thermodynamically stable and optically transparent or opaque depending on the particle size of the ernulsion. Microemulsions have a mean droplet size of less than 200 nm, in general between 20-100 nm. In contrast to conventional emulsions, the microemulsions are formed in the presence of an aqueous phase by self emulsification without any energy input. In the absence of water, this self emulsifying system exists as a transparent-looking mixture of oil and surfactants in which a lipophilic drug is dissolved.

Wheeler et al describe an emulsion preparation (U.S. patent 5,478,860) containing a mixture of paclitaxel, an oil and a polyethylene glycol-linked lipid which is covered by a monolayer of a polar lipid such as phosphatidylglycerol or phosphatidylethanolamine. This mixture, after homogenization in presence of an aqueous phase at appropriate pressure, yields an emulsion with a particle size in the range of 100 nm. It is not known if this is the mean or minimum particle size and if it is number weighted or volume weighted. The necessity of using undesirable organic solvents for initial dissolution of ingredients is not advisable even if the organic solvent is removed prior to use. In addition to an elaborate evaporation step, the method requires input of energy by way of high pressure homogenization adding to the overall cost. Because the preconcentrate of a true microemulsion is usually non-aqueous, it can provide longer shelf life than a regular emulsion which is in aqueous suspension.

Lacy et al disclose a capsule delivery system (U.S. patent 5,645,856) for oral delivery of hydrophobic drugs containing a digestible oil, and a combination of surfactants. The selection of surfactant is made such that it inhibits the in vivo lipolysis of the oil.

Eugster discloses an ultra microemulsion system (Swiss Patent CH 688 504 A5) for paclitaxel and its analogs composed of an oil and one or more surfactants providing a formulation of the drug with a mean particle size of 2.2-3 nm thus approaching a solution rather than an emulsion. It is not known if this formulation is useful for oral, injectable or topical use.

There have been attempts to enhance oral activity of taxanes by co-administration of taxanes with another drug such as cinchonine (WO 97/27855) or cyclosporin, ketoconazole etc. (WO 97/15269). Similarly, WO 97/48689 describes the use of various carbocyclic compounds in combination with anticancer drugs to enhance oral bioavailability of the drug. All three of these approaches have the drawback of combination drug therapy where a second drug with drastically different pharmacological activity is administered. In practice such a drug combination approach is the last resort taken by those familiar with the drug development process due to drastic increase in preclinical and clinical regulatory requirement for approval resulting in increasing cost and time to market.

### SUMMARY OF THE INVENTION

In accordance with the present invention it has now surprisingly been found that particularly stable anticancer drug formulations, particularly the taxanes, that self emulsify in aqueous medium giving an average particle size in a range of about .1 0 nm to about 10 microns .' and that have improved bioavailability characteristics, are obtainable. Also described are self-emulsifying preconcentrates that disperse, without the input of high energy (i.e., other than mixing energy to cause dispersion), to form droplets of average size of up to about 10 microns.

Accordingly, this invention provides a storage-stable, self-emulsifying preconcentrate of a taxane in a microemulsion, composed of:
(i) 10 to 80% w/w of at least one hydrophobic component selected from triglyceride, diglyceride, monoglyceride, free fatty acid, fatty acid ester, fish oil, vegetable oil, or mixtures thereof;
(ii) 20 to 80% w/w of a surfactant phase comprising at least one non-ionic surfactant;
(iii) 0 to 35% w/w of diethylene glycol monoethylether; and
(iv) 0 to 40% w/w of at least one hydrophilic component selected from a hydroxyalkane, dihydroxyalkane, a polyethylene glycol having an average molecular weight of less than or equal to 1000, and mixtures thereof; said preconcentrate producing an average particle size of less than or equal to 10 µm when mixed with an aqueous medium. Preferred embodiments of the invention are as defined in the attached claims.

Also provided is an aqueous taxane having an average particle size of less than or equal to 10µm, which comprises a storage-stable, self-emulsifying preconcentrate of a taxane in a microemulsion diluted in an aqueous medium, the preconcentrate comprising:
(a) 10 to 80% w/w of at least one hydrophobic component selected from triglyceride, diglyceride, monoglyceride, free fatty acid, fatty acid ester, fish oil, vegetable oil, or mixtures thereof;
(b) 20 to 80% w/w of a surfactant phase comprising at least one non-ionic surfactant;
(c) 0 to 35% w/w diethylene glycol monoethylether; and
(d) 0 to 40% w/w of at least one hydrophilic component selected from a hydroxyalkane, dihydroxyalkane, a polyethylene glycol having an average molecular weight of less than or equal to 1000, and mixtures thereof; said preconcentrate producing an average particle size of less than or equal to 10 µm when mixed with an aqueous medium.

Examples of suitable surfactants are:
1. Polyoxyethylene-sorbitan-fatty acid esters; e.g. mono- and tri-lauryl, palmityl, stearyl and oleyl esters; e.g. products of the type known as polysorbates and commercially available under the trade name "Tween".
2. Polyoxyethylene fatty acid esters, e.g., polyoxyethylene stearic acid esters of the type known and commercially available under the trade name Myrj.
3. Polyoxyethylene castor oil derivatives, e.g., products of the type known and commercially available as Cremophors® . Particularly suitable are polyoxyl 35 castor oil (Cremophor® EL) and polyoxyl 40 hydrogenated castor oil (Cremophor® RH40).
4. α-tocopherol, α-tocopheryl polyethylene glycol succinate (vitamin E TPGS), α-tocopherol palmitate and α-tocopherol acetate.
5. PEG glyceryl fatty acid esters such as PEG-8 glyceryl caprylate/caprate (commercially known as Labrasol), PEG-4 glyceryl caprylate/caprate (Labrafac Hydro WL 1219), PEG-32 glyceryl laurate (Gelucire 44/14), PEG-6 glyceryl mono oleate (Labrafil M 1944 CS), PEG-6 glyceryl linoleate (Labrafil M2125 CS).
6. Propylene glycol mono- and di-fatty acid esters, such as propylene glycol laurate, propylene glycol caprylate/caprate; also diethyleneglycol-monoethylether (DGME), commercially known as Transcutol (Gattefosse, Westwood, NJ).
7. Sorbitan fatty acid esters, such as the type known and commercially available under the name Span (e.g., Span 20).
8. Polyoxyethylene-polyoxypropylene co-polymers, e.g., products of the type known and commercially available as Pluronic or Poloxamer.
9. Glycerol triacetate.
10. Monoglycerides and acetylated monoglycerides, e.g., glycerol monodicocoate (Imwitor 928), glycerol monocaprylate (Imwitor 308), and mono-and di-acetylated monoglycerides.

Suitable surfactants are not limited to those mentioned above, but may include any compound or compounds that would enhance the galenic properties of the preconcentrate.

Compositions in accordance with the present invention may include other ingredients in addition to the drug, one or more hydrophobic components, one or more hydrophilic components, one or more surfactants, inhibitors of cytochrome P450 enzymes or inhibitors of the p-glycoprotein transport system such as grapefruit extract or compounds isolated from it. The composition may include, in addition to the forgoing, one or more ingredients, additives or diluents such as pharmaceutically acceptable polymeric or inorganic materials, anti-oxidants, preserving agents, flavoring orsweetening agents and so forth.

Compositions in accordance with the present invention may be liquid or solids at ambient temperature. They may be filled in soft or hard gelatin capsules in the form of liquid composition, molten composition, or granules or powder (if composition is solid at ambient temperature and was cooled and processed before filling). Coating may be also applied to capsules or tablets. The preconcentrate may be also be diluted with water to obtain stable emulsions that may be employed as drinking formulations, or packaged as such for injection after appropriate dilution with an aqueous medium, for example.

### DETAILED DESCRIPTION OF THE INVENTION

A self-emulsifying preconcentrate of the present invention comprising an anticancer drug must contain a hydrophobic component, a surfactant and optionally a hydrophilic component. The surfactant and hydrophilic component are needed for the composition to form in aqueous medium a self-emulsifying system having an average particle size of between about with an aqueous medium, for example.

### DETAILED DESCRIPTION OF THE INVENTION

A self-emulsifying preconcentrate of the present invention comprising a taxane as defined herein must contain a hydrophobic component, a surfactant and a hydrophilic component. The surfactant and hydrophilic component are needed for the composition to form in aqueous medium a self-emulsifying system having an average particle size of between about 10 nm and about 10 microns, They may also help enhance the solubility and stability of the anticancer drug in the formulation. The hydrophobic component is needed because if it is not incorporated in appropriate amounts in the formulation, precipitation of the drug will be observed upon mixing of the composition with an aqueous medium and/or on storage. Similar observations may be made for the hydrophilic and surfactant components.

Based on the above, appropriate combinations or mixtures of a hydrophobic component, a surfactant and a hydrophilic component with the water insoluble drug taxane are necessary to obtain a stable microemulsion preconcentrate that would yield upon mixing with an aqueous medium a stable dispersion with an average particle size of between about 10 nm and about 10 rnicrons.

Preferred as hydrophobic components are triglycerides, diglycerides, monoglycerides, free fatty acids, and fatty acid esters and derivatives thereof, individually or in combination. Examples of hydrophobic components include propylene glycol dicaprylate/caprate, caprilic/capric triglyceride, caprylic/capric/linoleic triglyceride, e.g. synthetic medium chain triglycerides having C8-12 fatty acid chains or other derivatized (synthetic) triglycerides of the type known and commercially available under Miglyol 810, 812, 818, 829 and 840, linoleic acid, linoleic acid ethyl ester, fish oils as free fatty acids, their esterification and their transesterification products, e.g. of the type known and commercially available under EPAX 6000 FA, EPAX 4510 TG, individually or in combination. Additional examples include vegetable oils and C12-18 fatty acid mono-, di- and triglycerides prepared by individual adrnixing or as transesterification products of vegetable oils (such as soybean oil, almond oil, sunflower oil, olive oil or corn oil) with glycerol.

Preferred as hydrophilic components are 1,2-propylene glycol, ethanol and polyethylene glycol having an average molecular weight of less than or equal to 1000, individually or in combination. More preferred as hydrophilic components are 1,2-propylene glycol and ethanol, individually or in combination. Especially preferred as hydrophilic components is a combination or mixture of 1,2-propylene glycol and ethanol.

The relative proportion of the drug and the other ingredients in the composition of the current invention will vary depending whether it is delivered as a self-emulsifying preconcentrate or after dilution with water, depending on the particular ingredients and the desired physical properties of the formulation. Concentration limits in the self-emulsifying preconcentrate are as follows:
1. Oil phase: from 10 to 80% w/w of the preconcentrate. The oil phase may consist of triglycerides, diglycerides, monoglycerides, free fatty acids, propylene glycol mono or diesters and free fatty acids, esters and derivatives thereof, individually or in combination.
2. Cumulative amounts of surfactants: from 20 to 80% w/w of the preconcentrate.
3. Cumulative amounts of hydrophilic components, such as 1,2-propylene glycol and/or ethanol and/or a polyethylene glycol having an average molecular weight of less than or equal to 1000 : from 0% to 40% w/w of the preconcentrate. The total of all ingredients will be 100%.

It is understood that the application of the teachings of the present invention, to the conditions described, will be evident to one skilled in the art of preparing such formulations.

In the following examples, the ingredients were weighed out into appropriate containers in the amounts described below. In all examples described below, a clear liquid was obtained upon appropriate mixing and heating.

### EXAMPLES

The formulations represented in the following examples were prepared by mixing the oil components with surfactants and cosurfactants followed by the addition of drug powder as indicated. The composition may be prepared at room temperature or heated to 40-50°C to accelerate the solubilization process. Several mixing techniques can be used ranging from mechanical stirring and agitation to sonication. All compositions shown below give liquid or semi-solid preconcentrates at room temperature.

An experiment to test the efficiency of forming microemulsions from the preconcentrates was carried out by diluting the preconcentrate in 20-50 fold with water or simulated gastric fluid with gentle mixing or shaking. The aqueous medium temperature varied between 20 and 37°C. Particle size analysis was then carried out using a photon correlation spectroscopy based particle sizer, Nicomp 370. Data reported in the following examples correspond to volume weighted panicle size.

### EXAMPLE 1

| Ingredients | Amount (g) |
|---|---|
| Miglyol 840 | 1.971 |
| Cremophor® RH40 | 2.190 |
| Imwitor 308 | 0.767 |
| Labrasol | 0.548 |
| Paclitaxel | 0.175 |
| Total | 5.651 |
| Mean particle size: 31 nm | |

### EXAMPLE 2

| Ingredients | Amount (g) |
|---|---|
| Miglyol 840 | 4.820 |
| Cremophor® RH40 | 4.990 |
| Imwitor 308 | 1.750 |
| Labrasol | 1.250 |
| Paclitaxel | 0.489 |
| Transcutol | 2.000 |
| Total | 15.299 |
| Mean particle size: 13 nm | |

### EXAMPLE 3

| Ingredients | Amount (g) |
|---|---|
| Miglyol 840 | 1.396 |
| Cremophor® RH40 | 1.551 |
| Imwitor 308 | 0.543 |
| Labrasol | 0.388 |
| Paclitaxel | 0.122 |
| Grapefruit extract | 0.400 |
| Total | 4.400 |
| Mean particle size: 30 nm. | |

### EXAMPLE 4

| Ingredients | Amount (g) |
|---|---|
| Miglyol 840 | 1.560 |
| Cremophor® RH40 | 1.610 |
| Imwitor 308 | 0.565 |
| Labrasol | 0.405 |
| Paclitaxel | 0.285 |
| Ethanol | 0.575 |
| Total | 5.000 |
| Mean particle size: 14nm | |

### EXAMPLE 5

| Ingredients | Amount (g) |
|---|---|
| Miglyol 812 | 1.435 |
| Tween 80 | 2.150 |
| Lipoid E80 | 0.705 |
| Soybean oil | 0.178 |
| Linoleic acid | 0.174 |
| Ethanol | 0.305 |
| Paclitaxel | 0.068 |
| Total | 5.000 |
| Mean particle size: 102 nm | |

### EXAMPLE 6

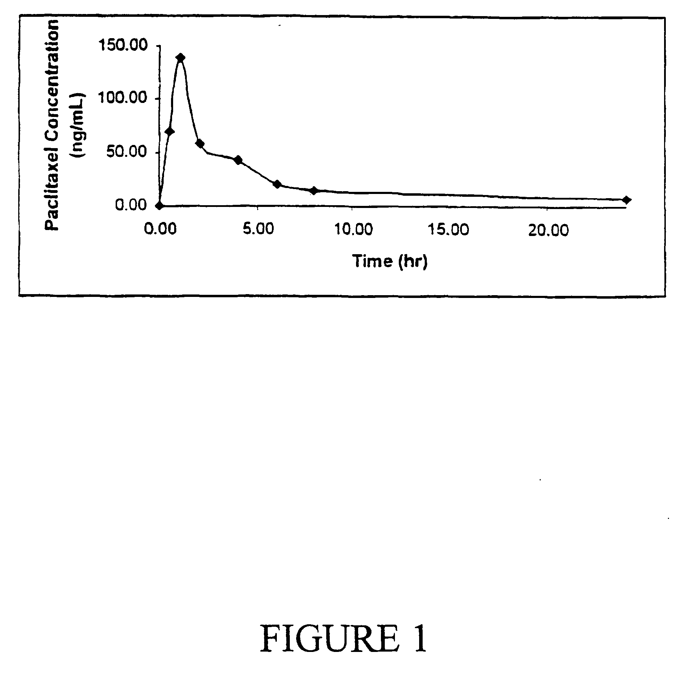

Bioavailability of paclitaxel micro-emulsion preconcentrate was assessed using the formulation described in Example 1. Paclitaxel was given in doses of 2.5 mg/kg or 5 mg/kg to 8 male dogs of approximately 10 kg body weight. The formulation was administered in thc morning after overnight fasting in the form of a capsule followed by water. Free access to food and water was allowed two hours after dosing. Blood samples were drawn at different point (pre-dose, 0.5, 1, 2, 3, 4, 6, 8, 12, and 24 hr) and stabilized with EDTA, placed in Vacutainers, and stored at 2-8°C. The blood samples were then extracted using a liquid-liquid method and assayed by HPLC/UV. Bioavailability calculations were done by comparing the pharmacokinetic (PK) profiles obtained for orally given paclitaxel micro-emulsion preconcentrate with an intravenous commercial formulation. Bioavailability values ranging from 25 % to 60 % were obtained. Figure 1 corresponds to a typical pharmacokinetic profile obtained for paclitaxel preconcentrate.

## Claims

1. A storage-stable, self-emulsifying preconcentrate of a taxane in a microemulsion, composed of:
(i) 10 to 80% w/w of at least one hydrophobic component selected from triglyceride, diglyceride, monoglyceride, free fatty acid, fatty acid ester, fish oil, vegetable oil, or mixtures thereof;
(ii) 20 to 80% w/w of a surfactant phase comprising at least one non-iqnic surfactant;
(iii) 0 to 35% w/w of diethylene glycol monoethylether; and
(iv) 0 to 40% w/w of at least one hydrophilic component selected from a hydroxyalkane, dihydroxyalkane, a polyethylene glycol having an average molecular weight of less than or equal to 1000, and mixtures thereof; said preconcentrate producing an average particle size of less than or equal to 10 µm when mixed with an aqueous medium.

2. The preconcentrate according to claim 1, wherein the surfactant comprises one or more non-ionic surfactants.

3. The preconcentrate according to claim 1, further including an inhibitor of P-glycoprotein transport system or an inhibitor of P450 enzymes.

4. The preconcentrate according to claim 3, wherein the inhibitor is a grapefruit extract or a component thereof.

5. The preconcentrate according to claims 1 or 4, wherein the carrier system comprises from 15 to 75% w/w of hydrophobic component.

6. The preconcentrate according to claims 1 or 5, wherein the carrier system comprises less than or equal to 30% w/w of hydrophilic component.

7. The preconcentrate according to claims 1 or 6, wherein the hydrophilic component is selected from the group consisting of 1,2-propylene glycol, ethanol, a polyethylene glycol and mixtures thereof.

8. The preconcentrate according to claims 1 or 7, wherein the hydrophilic component comprises a mixture of 1,2-propylene glycol and ethanol.

9. The preconcentrate according to claim 1 , wherein the hydrophobic component is selected from the group consisting of triglyceride, diglyceride, monoglyceride, free fatty acid, fatty acid ester and mixtures thereof.

10. The preconcentrate according to claim 1, wherein the hydrophilic component is selected from the group consisting of a fatty acid ester of a hydroxyalkane, a fatty acid ester of a dihydroxyalkane, a fatty acid mono-, di- or triglyceride or a transesterification product of a vegetable oil with a glycerol and mixtures thereof.

11. The preconcentrate according to claim 1, wherein the aqueous medium is water or simulated gastric fluid.

12. An orally administrable pharmaceutical composition comprising the preconcentrate according to claim 1, in a pharmaceutically acceptable diluent.

13. A parenterally injectable pharmaceutical composition comprising the preconcentrate according to claim 1, in a pharmaceutically acceptable diluent.

14. A preconcentrate according to claim 1 for oral or parenteral administration.

15. A preconcentrate according to claim 1 for enhancement of oral bioavailability of a taxane.

16. An aqueous taxane having an average particle size of less than or equal to 10µm, which comprises a storage-stable, self-emulsifying preconcentrate of a taxane in a microemulsion diluted in an aqueous medium, the preconcentrate comprising:
(a) 10 to 80% w/w of at least one hydrophobic component selected from triglyceride, diglyceride, monoglyceride, free fatty acid, fatty acid ester, fish oil, vegetable oil, or mixtures thereof;
(b) 20 to 80% w/w of a surfactant phase comprising at least one non-ionic surfactant;
(c) 0 to 35% w/w diethylene glycol monoethylether; and
(d) 0 to 40% w/w of at least one hydrophilic component selected from a hydroxyalkane, dihydroxyalkane, a polyethylene glycol having an average molecular weight of less than or equal to 1000, and mixtures thereof; said preconcentrate producing an average particle size of less than or equal to 10 µm when mixed with an aqueous medium.

## Patentansprüche

1. Lagerungsstabiles, selbstemulgierendes Präkonzentrat eines Taxans in einer Mikroemulsion, bestehend aus:
(i) 10 bis 80 Gew.-/Gew.-% von zumindest einer hydrophoben Komponente ausgewählt aus Triglycerid, Diglycerid, Monoglycerid, freier Fettsäure, Fettsäureester, Fischöl, Pflanzenöl oder Mischungen davon;
(ii) 20 bis 80 Gew.-Gew.-% einer Phase einer oberflächenaktiven Substanz, welche zumindest eine nichtionische oberflächenaktive Substanz aufweist;
(iii) 0 bis 35 Gew.-/Gew.-% Diethylenglycolmonoethylether; und
(iv) 0 bis 40 Gew.-/Gew.-% von zumindest einer hydrophilen Komponente ausgewählt aus einem Hydroxyalkan, Dihydroxyalkan, einem Polyethylenglycol mit einem durchschnittlichen relativen Molekulargewicht von weniger als oder gleich 1000 und Mischungen davon, wobei das Präkonzentrat bei Mischung mit einem wässrigen Medium eine durchschnittliche Partikelgröße von weniger als oder gleich 10 µm ergibt.

2. Präkonzentrat nach Anspruch 1, wobei die oberflächenaktive Substanz eine oder mehrere nichtionische oberflächenaktive Substanzen umfasst.

3. Präkonzentrat nach Anspruch 1, welches ferner einen Inhibitor des P-Glycoprotein-Transportsystems oder einen Inhibitor von P450-Enzymen beinhaltet.

4. Präkonzentrat nach Anspruch 3, wobei der Inhibitor ein Grapefruitextrakt oder eine Komponente davon ist.

5. Präkonzentrat nach Anspruch 1 oder 4, wobei das Trägersystem zwischen 15 und 75 Gew.-/Gew.-% einer hydrophoben Komponente aufweist.

6. Präkonzentrat nach Anspruch 1 oder 5, wobei das Trägersystem weniger als oder gleich 30 Gew.-/Gew.-% einer hydrophilen Komponente aufweist.

7. Präkonzentrat nach Anspruch 1 oder 6, wobei die hydrophile Komponente ausgewählt ist aus der Gruppe bestehend aus 1,2-Propylenglycol, Ethanol, einem Polyethylenglycol und Mischungen davon.

8. Präkonzentrat nach Anspruch 1 oder 7, wobei die hydrophile Komponente eine Mischung aus 1,2-Propylenglycol und Ethanol aufweist.

9. Präkonzentrat nach Anspruch 1, wobei die hydrophobe Komponente ausgewählt ist aus der Gruppe bestehend aus Triglycerid, Diglycerid, Monoglycerid, freier Fettsäure, Fettsäureester und Mischungen davon.

10. Präkonzentrat nach Anspruch 1, wobei die hydrophile Komponente ausgewählt ist aus der Gruppe bestehend aus einem Fettsäureester eines Hydroxyalkans, einem Fettsäureester eines Dihydroxyalkans, einem Fettsäure-Mono-, -Di- oder -Triglycerid oder einem Umesterungsprodukt eines Pflanzenöls mit einem Glycerol und Mischungen davon.

11. Präkonzentrat nach Anspruch 1, wobei das wässrige Medium Wasser oder simulierte Magenflüssigkeit ist.

12. Oral verabreichbare pharmazeutische Zusammensetzung, welche das Präkonzentrat nach Anspruch 1, in einem pharmazeutisch annehmbaren Verdünnungsmittel aufweist.

13. Parenteral injizierbare pharmazeutische Zusammensetzung, welche das Präkonzentrat nach Anspruch 1, in einem pharmazeutisch annehmbaren Verdünnungsmittel aufweist.

14. Präkonzentrat nach Anspruch 1 für die orale oder parenterale Verabreichung.

15. Präkonzentrat nach Anspruch 1 für die Verbesserung der oralen Bioverfügbarkeit eines Taxans.

16. Wässriges Taxan mit einer durchschnittlichen Partikelgröße von weniger als oder gleich 10 µm, welches ein lagerungsstabiles, selbstemulgierendes Präkonzentrat eines Taxans in einer Mikroemulsion verdünnt in einem wässrigen Medium aufweist, wobei das Präkonzentrat folgendes enthält:
(a) 10 bis 80 Gew.-/Gew.-% von zumindest einer hydrophoben Komponente ausgewählt aus Triglycerid, Diglycerid, Monoglycerid, freier Fettsäure, Fettsäureester, Fischöl, Pflanzenöl oder Mischungen davon; .
(b) 20 bis 80 Gew.-/Gew.-% einer Phase einer oberflächenaktiven Substanz, welche zumindest eine nichtionische oberflächenaktive Substanz aufweist;
(c) 0 bis 35 Gew.-/Gew.-% Diethylenglycolmonoethylether; und
(d) 0 bis 40 Gew.-/Gew.-% von zumindest einer hydrophilen Komponente ausgewählt aus einem Hydroxyalkan, Dihydroxyalkan, einem Polyethylenglycol mit einer durchschnittlichen relativen Molekulargewicht von weniger als oder gleich 1000 und Mischungen davon, wobei das Präkonzentrat bei Mischung mit einem wässrigen Medium eine durchschnittliche Partikelgröße von weniger als oder gleich 10 µm ergibt.

## Revendications

1. Préconcentré stable au stockage, auto-émulsifiant d'un taxane dans une microémulsion, composé de :
(i) 10 à 80% p/p d'au moins un composant hydrophobe choisi parmi un triglycéride, un diglycéride, un monoglycéride, un acide gras libre, un ester d'acide gras, une huile de poisson, une huile végétale ou des mélanges de ceux-ci ;
(ii) 20 à 80% p/p d'une phase tensioactive comprenant au moins un tensioactif non ionique ;
(iii) 0 à 35% p/p de monométhyléther de diéthylèneglycol ; et
(iv) 0 à 40% p/p d'au moins un composant hydrophile choisi parmi un hydroxyalcane, un dihydroxyalcane, un polyéthylèneglycol ayant un poids moléculaire moyen inférieur ou égal à 1000 et des mélanges de ceux-ci ; ledit préconcentré produisant une taille moyenne des particules inférieure ou égale à 10 µm quand il est mélangé avec un milieu aqueux.

2. Préconcentré selon la revendication 1, dans lequel le tensioactif comprend un ou plusieurs tensioactifs non ioniques.

3. Préconcentré selon la revendication 1, incluant en outre un inhibiteur du système de transport de P-glycoprotéine ou un inhibiteur des enzymes P450.

4. Préconcentré selon la revendication 3, dans lequel l'inhibiteur est un extrait de pamplemousse ou un composant de celui-ci.

5. Préconcentré selon les revendications 1 ou 4, dans lequel le système support comprend de 15 à 75% p/p d'un composant hydrophobe.

6. Préconcentré selon les revendications 1 ou 5, dans lequel le système support comprend une quantité inférieure ou égale à 30% p/p de composant hydrophile.

7. Préconcentré selon les revendications 1 ou 6, dans lequel le composant hydrophile est choisi parmi le groupe constitué du 1,2-propylèneglycol, de l'éthanol, d'un polyéthylèneglycol et des mélanges de ceux-ci.

8. Préconcentré selon les revendications 1 ou 7, dans lequel le composant hydrophile comprend un mélange de 1,2-propylèneglycol et d'éthanol.

9. Préconcentré selon la revendication 1, dans lequel le composant hydrophobe est choisi parmi le groupe constitué d'un triglycéride, d'un diglycéride, d'un monoglycéride, d'un acide gras libre, d'un ester d'acide gras et des mélanges de ceux-ci.

10. Préconcentré selon la revendication 1, dans lequel le composant hydrophile est choisi parmi le groupe constitué d'un ester d'acide gras d'un hydroxyalcane, d'un ester d'acide gras d'un dihydroxyalcane, d'un mono-, di- ou triglycéride d'acide gras ou d'un produit de la transestérification d'une huile végétale avec un glycérol et des mélanges de ceux-ci.

11. Préconcentré selon la revendication 1, dans lequel le milieu aqueux est de l'eau ou un fluide gastrique simulé.

12. Composition pharmaceutique administrable par voie orale comprenant le préconcentré selon la revendication 1, dans un diluant pharmaceutiquement acceptable.

13. Composition pharmaceutique injectable par voie parentérale comprenant le préconcentré selon la revendication 1, dans un diluant pharmaceutiquement acceptable.

14. Préconcentré selon la revendication 1 pour l'administration orale ou parentérale.

15. Préconcentré selon la revendication 1 pour l'amélioration de la biodisponibilité orale d'un taxane.

16. Taxane aqueux ayant une taille moyenne des particules inférieure ou égale à 10 µm, qui comprend un préconcentré stable au stockage, auto-émulsifiant d'un taxane dans une microémulsion diluée dans un milieu aqueux, le préconcentré comprenant :
(a) 10 à 80% p/p d'au moins un composant hydrophobe choisi parmi un triglycéride, un diglycéride, un monoglycéride, un acide gras libre, un ester d'acide gras, une huile de poisson, une huile végétale ou des mélanges de ceux-ci ;
(b) 20 à 80% p/p d'une phase tensioactive comprenant au moins un tensioactif non ionique ;
(c) 0 à 35% p/p de monométhyléther de diéthylèneglycol ; et
(d) 0 à 40% p/p d'au moins un composant hydrophile choisi parmi un hydroxyalcane, un dihydroxyalcane, un polyéthylèneglycol ayant un poids moléculaire moyen inférieur ou égal à 1000 et des mélanges de ceux-ci ; ledit préconcentré produisant une taille moyenne des particules inférieure ou égale à 10 µm quand il est mélangé avec un milieu aqueux.
